# EUROPÄISCHE PATENTSCHRIFT

(11) **EP 3 233 025 B1**
(45) Veröffentlichungstag und Bekanntmachung des Hinweises auf die Patenterteilung: **01.01.2020**
(21) Anmeldenummer: 15793838.2
(22) Anmeldetag: 12.11.2015
(51) Int. Cl.: A61K 8/26, A61K 8/27, A61K 8/28, A61K 8/29, A61K 8/365, A61Q 5/00, A61Q 5/02, A61K 8/64

(54) **HAARBEHANDLUNGSMITTEL, ENTHALTEND MINDESTENS EIN SAURES PROTEIN SOWIE MINDESTENS EIN SALZ**
COMPOSITION FOR TREATING HAIR CONTAINING AN ACIDIC PROTEIN AND AT LEAST A SALT
COMPOSITION POUR LE TRAITEMENT DES CHEVEUX CONTENANT UN PROTEINE ACIDIQUE ET AU MOINS UN SEL

(30) Priorität: 17.12.2014 DE 102014226175
(43) Veröffentlichungstag der Anmeldung: 25.10.2017
(73) Patentinhaber: Henkel AG & Co. KGaA, 40589 Düsseldorf (DE)
(72) Erfinder: SCHULZE ZUR WIESCHE, Erik, 22453 Hamburg (DE); KROHN, René, 22851 Norderstedt (DE)
(86) Internationale Anmeldenummer: PCT/EP2015/076436
(87) Internationale Veröffentlichungsnummer: WO 2016/096267

(56) Entgegenhaltungen:
- EP-A1- 1 325 736
- WO-A1-2009/106399
- DE-A1- 10 051 773
- DE-A1-102005 061 022
- J. H. WOYCHIK ET AL: "Wheat Gluten Proteins, Amino Acid Composition of Proteins in Wheat Gluten", JOURNAL OF AGRICULTURAL AND FOOD CHEMISTRY, Bd. 9, Nr. 4, 1. Juli 1961 (1961-07-01), Seiten 307-310, XP055035404, ISSN: 0021-8561, DOI: 10.1021/jf60116a020
- By K A Kuiken ET AL: "ESSENTIAL AMINO ACID COMPOSITION OF SOY BEAN MEALS PREPARED FROM TWENTY STRAINS OF SOY BEANS*", , 1. Januar 1948 (1948-01-01), XP055235278, Gefunden im Internet: URL:http://www.jbc.org/content/177/1/29.fu ll.pdf [gefunden am 2015-12-10]
- Pomeranz: "Protein Content and Amino Acid Composition of Oat Species and Tissues", , 1. Januar 1973 (1973-01-01), Seiten 702-707, XP055235280, Gefunden im Internet: URL:http://www.aaccnet.org/publications/cc /backissues/1973/Documents/chem50_702.pdf [gefunden am 2015-12-10]
- Anonymous: "GNPD - Shampoo", , 1. Juni 2007 (2007-06-01), XP055235282, Gefunden im Internet: URL:http://www.gnpd.com/sinatra/recordpage /717909/from_search/HZoF2gVfAJ/ [gefunden am 2015-12-10]

## Beschreibung

Die vorliegende Erfindung liegt auf dem Gebiet der Kosmetik und betrifft ein kosmetisches Mittel, welches mindestens ein saures Protein sowie mindestens ein Salz, umfassend mindestens ein zweiwertiges Kation und/oder mindestens ein Salz, umfassend mindestens ein dreiwertiges Kation, enthalt.

Weiterhin betrifft die vorliegende Erfindung ein Verfahren zum Farberhalt gefärbter keratinischer Fasern unter Verwendung des erfindungsgemäßen kosmetischen Mittels.

Schließlich betrifft die vorliegende Erfindung die Verwendung einer Kombination aus mindestens einem sauren Protein und mindestens einem Salz, enthaltend mindestens ein zweiwertiges Kation und/oder mindestens einem Salz, enthaltend mindestens ein dreiwertiges Kation, zum Farberhalt gefärbter keratinischer Fasern.

Die Veränderung der Form und der Farbe von Haaren stellt einen wichtigen Bereich der modernen Kosmetik dar. Dadurch kann das Erscheinungsbild der Haare sowohl an aktuelle Modeströmungen als auch an die individuellen Wünsche des einzelnen Verbrauchers angepasst werden. Die modische Farbgestaltung von Frisuren oder die Kaschierung von ergrautem oder weißem Haar mit modischen oder natürlichen Farbtönen erfolgt üblicherweise mit farbverändernden Mitteln, welche die Haare permanent oder nur vorübergehend, d.h. temporär, färben.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus. Die Oxidationsfärbemittel zeichnen sich durch hervorragende, lang anhaltende Färbeergebnisse aus.

Für temporäre Färbungen werden üblicherweise Färbe- oder Tönungsmittel verwendet, welche als färbende Komponente sogenannte Direktzieher enthalten. Hierbei handelt es sich um Farbstoffmoleküle, welche direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Im Gegensatz zu den mit Oxidationsfärbemittel erhältliche Färbungen weise die Färbeergebnisse temporärer Färbungen eine geringere Haltbarkeit auf.

Die mit den zuvor beschriebenen Färbesystemen gefärbten keratinischen Fasern, insbesondere Haare, weisen jedoch den Nachteil auf, dass sie sich unter äußeren Einflüssen - beispielsweise während oder nach der Haarreinigung - in unerwünschter Weise verändern können.

Unter "unerwünschter Veränderung" wird das Verblassen oder Ausbluten, der Verlust der Farbbrillanz sowie die Farbverschiebung des durch die jeweilige Färbung erzielten Farbtons der Haare verstanden. Eine unerwünschte Veränderung der Haarfarbe tritt in der Regel während oder nach der Haarreinigung auf. Der Kontakt der Haare mit Wasser und Tensiden, aber auch das Einmassieren des Shampoos, das Abtrocknen der Haare nach dem Ausspülen des Shampoos oder die Fönhitze beim anschließenden Trocknungsvorgang können die Haftung der Haarfarbe beeinträchtigen und zu einer unerwünschten Farbveränderung und/oder zu weniger Brillanz der Haarfarbe führen. Durch weitere Umwelteinflüsse und/oder Sonneneinwirkungen kann die hervorgerufene unerwünschte Veränderung noch zusätzlich verstärkt werden.

Haarbehandlungsmittel, welche die unerwünschte Veränderung von gefärbten Haaren vermeiden und die Farbe der gefärbten Haare erhalten, sind beispielsweise in der Offenlegungsschrift EP 1 676 604 A1 beschrieben und enthalten neben einem anionischen Tensid und einem speziellen Silikon mindestens ein wasserlösliches Salz, bevorzugt Natriumsulfat.

DE 102005061022 A1 beschreibt die Verwendung von derivatisierten Proteinhydrolysaten zur Verbesserung der Farbstabilität von gefärbten Keratinfasern. Als Proteinhydrolysat kann beispielsweise Seidenprotein-Hydrolysat eingesetzt werden.

DE 10051773 A1 betrifft die Verwendung von kurzkettigen Carbonsäuren zur Farbstabilisierung von Färbungen gefärbter keratinischer Fasern. Diese Carbonsäuren können auch in Form ihrer Salze eingesetzt werden. Nach Lehre von DE 10051773 A1 können die Carbonsäuren mit Proteinhydrolysaten kombiniert werden.

In EP 1325736 A1 werden Emulsionen enthaltend modifizierte Polyglutaminsäurederivate beansprucht. In den Emulsionen können - als optionale Bestandteile - auch Salze wie Titandioxid, Zinkoxid, Bariumsulfat eingesetzt werden.

WO 2009/106399 A1 beschäftigt sich mit der kosmetischen Verwendung von Cranberry-Extrakt und nennt als weiteren möglichen Bestandteil auch Proteinhydrolysate.

Die im Stand der Technik bekannten Haarbehandlungsmittel führen jedoch nicht immer zu dem gewünschten Farberhalt, insbesondere der Verringerung der Farbverschiebung.

Der vorliegenden Erfindung lag daher die Aufgabe zugrunde, ein kosmetisches Mittel zur Behandlung keratinischer Fasern bereitzustellen, welches die Nachteile des Standes der Technik vermeidet bzw. zumindest abschwächt und welches zu einem verbesserten Farberhalt, insbesondere zu einer verminderten Farbverschiebung, führt. Neben dem verbesserten Farberhalt sollen die kosmetischen Mittel eine gute Reinigungs- bzw. Pflegeleistung sowie eine hohe Lagerstabilität aufweisen.

Es wurde nun überraschenderweise gefunden, dass der Einsatz von bestimmten sauren Proteinen in Kombination mit mindestens einem Salz in kosmetischen Mitteln zur Haarbehandlung zu einem verbesserten Farberhalt führt. Insbesondere die unerwünschte Verschiebung bzw. Veränderung der Haarfarbe in Richtung Gelb bzw. Blau kann durch den erfindungsgemäßen Einsatz der zuvor angeführten Kombination vermieden bzw. vermindert werden. Darüber hinaus führt die Verwendung des sauren Proteins in Kombination mit dem mindestens einen Salz nicht zu ungünstigen Wechselwirkungen mit weiteren Inhaltsstoffen der kosmetischen Mittel, so dass ein negativer Einfluss auf die Reinigungs- bzw. Pflegeleistung sowie die Lagerstabilität dieser Mittel vermieden wird.

Ein erster Gegenstand der Erfindung ist daher ein kosmetisches Mittel zur Behandlung keratinischer Fasern, enthaltend in einem kosmetisch verträglichen Träger - bezogen auf das Gesamtgewicht des kosmetischen Mittels -
a) 0,001 bis 10 Gew.-% mindestens eines Proteins, welches einen Gehalt an Glutaminsäure und/oder Asparaginsäure von 10 bis 60 %, bezogen auf den Gesamtgehalt aller Aminosäuren des Proteins, aufweist, und
b) 0,01 bis 10 Gew.-% mindestens eines Salzes, welches mindestens ein zweiwertiges Kation umfasst, und/oder mindestens eines Salzes, welches mindestens ein dreiwertiges Kation umfasst,
dadurch gekennzeichnet, dass als Salz, welches mindestens ein dreiwertiges Kation umfasst, ein Salz aus der Gruppe von Aluminiumsalzen der Formel MAl(SO₄)₂, worin M für ein Kalium-, Natrium-, Guanidinium- oder Ammoniumion steht, enthalten ist.

Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn die kosmetischen Mittel zur Behandlung von menschlichen Haaren eingesetzt werden.

Weiterhin werden unter dem Begriff "Protein" im Rahmen der vorliegenden Erfindung chemische Verbindungen, welche durch Peptid-Bindungen Säureamid-artig verknüpfte Kondensationsprodukte von Aminosäuren bilden, verstanden. Die Anzahl der Aminosäuren in den Proteinen beträgt bevorzugt mindestens 2 und maximal 1.000 Aminosäuren. Unter dem Begriff "Protein" sind erfindungsgemäß auch Hydrolysate eines Proteins zu verstehen, welche Proteinfraktionen mit verschiedenen Aminosäuresequenzen und Molekulargewichten enthalten.

Zudem werden unter dem Begriff "zweiwertige Kationen" im Rahmen der vorliegenden Erfindung Kationen verstanden, welche zweifach positiv geladen sind. Dahingegen werden unter dem Begriff "dreiwertige Kationen" Kationen verstanden, welche dreifach positiv geladen sind.

Darüber hinaus sind unter "kosmetischen Mittel zur Behandlung keratinischer Fasern" bevorzugt Haarreinigungsmittel wie Shampoos, Haarpflegemittel wie Haarkuren, Spülungen oder Haarpflegesprays sowie Haarstylingmittel wie Haargele, Haarsprays oder Haarwachse zu verstehen.

Weiterhin werden unter dem Begriff "Fettalkohole" im Rahmen der vorliegenden Erfindung aliphatische, langkettige, einwertige, primäre Alkohole verstanden, welche unverzweigte Kohlenwasserstoffreste mit 6 bis 30 Kohlenstoffatomen aufweisen. Die Kohlenwasserstoffreste können gesättigt aber auch ein- oder mehrfach ungesättigt sein.

Schließlich werden unter dem Begriff "Fettsäuren" im Rahmen der vorliegenden Erfindung aliphatische Monocarbonsäuren mit unverzweigter Kohlenstoffkette verstanden, welche Kohlenwasserstoffreste mit 6 bis 30 Kohlenstoffatomen aufweisen. Die Kohlenwasserstoffreste können entweder gesättigt oder auch ein- oder mehrfach ungesättigt sein.

Die Angabe der Gesamtmenge in Bezug auf die Komponenten des kosmetischen Mittels bezieht sich vorliegend - sofern nichts anderes angegeben ist - auf die Gesamtmenge an Aktivsubstanz der jeweiligen Komponente.

Die erfindungsgemäßen kosmetischen Mittel enthalten einen kosmetischen Träger. Erfindungsgemäß bevorzugt ist der kosmetische Träger wässrig, alkoholisch oder wässrig-alkoholisch. Im Rahmen der vorliegenden Erfindung können beispielsweise Cremes, Emulsionen, Gele oder tensidhaltige schäumende Lösungen, wie beispielsweise Shampoos, Schaumaerosole oder andere Zubereitungen, welche für die Anwendung auf dem Haar geeignet sind, eingesetzt werden.

Ein wässriger Träger im Sinne der Erfindung enthält mindestens 30 Gew.-%, insbesondere mindestens 50 Gew.-%, Wasser, bezogen auf das Gesamtgewicht des kosmetischen Mittels.

Unter wässrig-alkoholischen Trägern sind im Sinne der vorliegenden Erfindung wasserhaltige Zusammensetzungen, enthaltend einen C₁-C₄-Alkohol in einer Gesamtmenge von 3 bis 90 Gew.-%, bezogen auf das Gesamtgewicht des kosmetischen Mittels, insbesondere Ethanol bzw. Isopropanol, zu verstehen.

Die erfindungsgemäßen kosmetischen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des kosmetischen Mittels, enthalten ist.

Das erfindungsgemäße kosmetische Mittel enthält als ersten wesentlichen Bestandteil a) mindestens ein Protein, welches einen bestimmten Anteil an sauren Aminosäuren, insbesondere Glutaminsäure und/oder Asparaginsäure, enthält.

Erfindungsgemäß bevorzugt weist das mindestens eine Protein einen Gehalt an Glutaminsäure und/oder Asparaginsäure von 20 bis 60 %, vorzugsweise von 30 bis 60 %, insbesondere von 40 bis 60 %, bezogen auf den Gesamtgehalt aller Aminosäuren des Proteins, auf. Der Gehalt an Glutaminsäure und/oder Asparaginsäure in dem erfindungsgemäß eingesetzten Protein bezieht sich auf den prozentualen Anteil der zuvor genannten Aminosäuren an dem Gesamtgehalt, insbesondere der Gesamtmenge, aller in dem Protein vorkommenden Aminosäuren. Die Bestimmung des Gehalts an Glutaminsäure und/oder Asparaginsäure kann beispielsweise durch Hydrolyse des Proteins, anschließender Derivatisierung der Aminosäuren und nachfolgender Bestimmung der derivatisierten Aminosäuren mittels HPLC erfolgen (S. A. Cohen in "Quantification of amino acids and amines by chromatography - methods and protocols", Elsevier B.V., 2005, Seiten 242 bis 267). Proteine, welche den zuvor angeführten Gehalt an sauren Aminosäuren aufweisen, führen in Kombination mit dem mindestens einen Salz zu einem besonders guten Farberhalt bzw. zu einer besonders starken Verminderung der Farbverschiebung von gefärbten keratinischen Fasern, insbesondere Haaren.

Gemäß einer weiteren bevorzugten Ausführungsform weist das mindestens eine Protein einen pKₛ-Wert von 3,5 bis 6,5, vorzugsweise von 4,0 bis 6,0, bevorzugt von 4,5 bis 6,0, insbesondere von 4,5 bis 5,5, auf. Die Bestimmung der zuvor angeführten pKₛ-Werte von kann beispielsweise über eine Säure-Base-Titration unter Verwendung von dem Fachmann bekannten Indikatoren und der Bestimmung des pH-Werts am Halbäquivalenzpunkt ermittelt werden. An diesem Punkt liegen die Säure und ihre korrespondierende Base in gleicher Konzentration vor und der pKₛ-Wert entspricht dem pH-Wert. Der Einsatz von sauren Proteinen, welche die obigen pKs-Werte aufweisen, in Kombination mit dem mindestens einen Salz resultiert in besonders guten Ergebnissen in Bezug auf den Farberhalt gefärbter keratinischer Fasern.

Aufgrund der hohen Gesamtgehalts an sauren Aminosäuren weist das in den erfindungsgemäßen kosmetischen Mitteln eingesetzte Protein einen sauren pH-Wert auf. Bevorzugt ist es daher, wenn eine 1 Gew.-%ige Lösung des mindestens einen Proteins, bezogen auf das Gesamtgewicht der Lösung, einen pH-Wert, gemessen bei 20 °C, von pH 3,5 bis pH 7,0, vorzugsweise von pH 3,5 bis pH 6,0, insbesondere von pH 3,5 bis pH 5,2, aufweist. Unter Lösung des mindestens einen Proteins wird im Rahmen der vorliegenden Erfindung eine Lösung verstanden, in welcher 1 Gew.-% des Proteins in einem Lösungsmittel, bevorzugt Wasser, vollständig gelöst vorliegt.

Besonders gute Ergebnisse im Hinblick auf den Farberhalt, die Reinigungs- bzw. Pflegeleistung sowie die Lagerstabilität der erfindungsgemäßen kosmetischen Mittel werden erhalten, wenn das mindestens eine Protein ein mittleres Molekulargewicht M_{w} von 100 bis 5.000 Da, vorzugsweise von 100 bis 4.000 Da, bevorzugt von 200 bis 3.000 Da, insbesondere von 300 bis 1.200 Da, aufweist. Das mittlere Molekulargewicht M_{w} kann beispielsweise durch Gelpermeationschromatographie (GPC) bestimmt werden (Andrews P.; "Estimation of the Molecular Weights of Proteins by Sephadex Gel-Filtration"; Biochem. J., 1964, 91, Seiten 222 bis 233). Der Einsatz von sauren Proteinen, welche die zuvor genannten mittleren Molekulargewichte M_{w} enthalten, führt zum einen zu einem hervorragenden Farbschutz bei gleichzeitig hoher Reinigungs- bzw. Pflegeleistung sowie Lagerstabilität.

Das mindestens eine Protein enthält bevorzugt 2 bis 40 Aminosäuren, vorzugsweise aus 2 bis 30 Aminosäuren, bevorzugt aus 2 bis 25 Aminosäuren, insbesondere aus 2 bis 18 Aminosäuren. Proteine, welche die zuvor angeführte Anzahl an Aminosäuren aufweisen, haben sich im Rahmen der vorliegenden Erfindung im Hinblick auf den Farberhalt sowie die Reinigungs- bzw. Pflegeleistung als besonders vorteilhaft erwiesen.

Erfindungsgemäß bevorzugt ist das mindestens eine Protein in einer Gesamtmenge von 0,001 bis 8 Gew.-%, vorzugsweise von 0,01 bis 5 Gew.-%, bevorzugt von 0,1 bis 3 Gew.-%, insbesondere von 0,3 bis 2 Gew.-%, bezogen auf das Gesamtgewicht des kosmetischen Mittels, enthalten. Der Einsatz der zuvor angeführten Mengen des mindestens einen sauren Proteins in den erfindungsgemäßen kosmetischen Mitteln gewährleistet einerseits einen hervorragenden Farberhalt der gefärbten keratinischen Fasern und führt andererseits nicht zu negativen Wechselwirkungen mit weiteren Inhaltsstoffen der erfindungsgemäßen kosmetischen Mittel, so dass eine zufriedenstellende Reinigungs- bzw. Pflegeleistung sowie eine hohe Lagerstabilität dieser Mittel resultiert.

Als zweite wesentliche Komponente b) enthalten die erfindungsgemäßen kosmetischen Mittel 0,01 bis 10 Gew.-% mindestens eines Salzes, welches mindestens ein zweiwertiges Kation umfasst, und/oder mindestens eines Salzes, welches mindestens ein dreiwertiges Kation umfasst,
dadurch gekennzeichnet, dass als Salz, welches mindestens ein dreiwertiges Kation umfasst, ein Salz aus der Gruppe von Aluminiumsalzen der Formel MAl(SO₄)₂, worin M für ein Kalium-, Natrium-, Guanidinium- oder Ammoniumion steht, enthalten ist. Als Salze werden im Rahmen der vorliegenden Erfindung chemische Verbindungen bezeichnet, welche aus positiv geladenen Ionen (sogenannte Kationen) und negativ geladenen Ionen (sogenannte Anionen) aufgebaut sind. Hierunter fallen sowohl anorganische Salze, in welchen weder die Anionen noch die Kationen organische Verbindungen, d. h. auf Kohlenstoff basierende Verbindungen, darstellen, sowie organische Salze, bei welchen mindestens ein Anion oder ein Kation in Form einer organischen Verbindung vorliegt.

Besonders gute Effekte im Hinblick auf den Farbschutz, die Reinigungs- bzw. Pflegeleistung sowie die Lagerstabilität der erfindungsgemäßen kosmetischen Mittel werden erhalten, wenn als Salz, welches mindestens ein zweiwertiges Kation umfasst, ein Salz der Milchsäure, ausgewählt aus der Gruppe von Magnesiumsalzen, Calciumsalzen, Kupfersalzen, Zinksalzen, Eisen(II)-salzen sowie Mischungen dieser Salze, insbesondere Calciumsalzen der Milchsäure, enthalten ist. Im Rahmen der vorliegenden Erfindung ist der Einsatz von Calciumsalzen der Milchsäure, auch als Calciumlactat bezeichnet, besonders bevorzugt. Calciumlactat führt in Verbindung mit dem mindestens einen sauren Protein zu einem besonders guten Farbschutz sowie einer besonders hohen Verminderung der Farbverschiebung von gefärbten keratinischen Fasern. Darüber hinaus führt der Einsatz der zuvor angeführten Salze nicht zu einem negativen Einfluss auf die Reinigungs- bzw. Pflegeleistung der erfindungsgemäßen kosmetischen Mittel.

Bevorzugt ist das Salz, welches mindestens ein zweiwertiges Kation umfasst, in einer Gesamtmenge von 0,01 bis 8 Gew.-%, vorzugsweise von 0,1 bis 5 Gew.-%, bevorzugt von 0,2 bis 3 Gew.-%, insbesondere von 0,3 bis 2 Gew.-%, bezogen auf das Gesamtgewicht des kosmetischen Mittels, enthalten. Der Einsatz des mindestens einen Salzes, bevorzugt Calciumlactat, in den zuvor angeführten Mengen resultiert in einem besonders guten Farbschutz und führt nicht zu einer Ausfällung des sauren Proteins oder zu negativen Wechselwirkungen mit weiteren üblichen Inhaltsstoffen des erfindungsgemäßen kosmetischen Mittels, so dass eine hohe Lagerstabilität gewährleistet wird. Darüber hinaus führt der Einsatz dieser Salzmengen auch nicht zu einem negativen Einfluss auf die Reinigungs- bzw. Pflegeleistung der erfindungsgemäßen kosmetischen Mittel.

Im Rahmen der vorliegenden Erfindung wird ein Salz eingesetzt, welches mindestens ein dreiwertiges Kation umfasst, wobei als Salz, welches mindestens ein dreiwertiges Kation umfasst, ein Salz aus der Gruppe von Aluminiumsalzen der Formel MAl(SO4)₂, worin M für ein Kalium-, Natrium-, Guanidinium- oder Ammoniumion steht, enthalten ist. Im Rahmen der vorliegenden Erfindung ist der Einsatz von Aluminiumsalzen, insbesondere solchen der obigen Formel MAl(SO₄)₂ mit M = K⁺, welche als sogenannte Alaune bezeichnet werden, besonders bevorzugt. Der Einsatz von Alaunen führt in Kombination mit dem sauren Protein sowie gegebenenfalls mit dem weiteren Salz, insbesondere Calciumlactat, zu besonders guten Ergebnissen in Bezug auf den Farberhalt und die Lagerstabilität der erfindungsgemäßen kosmetischen Mittel.

Im Rahmen der vorliegenden Erfindung ist es bevorzugt, wenn das Salz, welches mindestens ein dreiwertiges Kation umfasst, in einer bestimmten Gesamtmenge in den erfindungsgemäßen kosmetischen Mittel eingesetzt wird. Erfindungsgemäß bevorzugte kosmetische Mittel sind daher dadurch gekennzeichnet, dass das Salz, welches mindestens ein dreiwertiges Kation umfasst, in einer Gesamtmenge von 0,01 bis 5 Gew.-%, vorzugsweise von 0,05 bis 4,5 Gew.-%, bevorzugt von 0,1 bis 4,0 Gew.-%, weiter bevorzugt von 0,15 bis 3,5 Gew.-%, noch weiter bevorzugt von 0,5 bis 3,0 Gew.-%, insbesondere von 0,8 bis 2,5 Gew.-%, bezogen auf das Gesamtgewicht des kosmetischen Mittels, enthalten ist. Der Einsatz des mindestens einen Salzes, bevorzugt eines Aluminiumsalzes der Formel Formel MAl(SO₄)₂ mit M = K⁺, in den zuvor angeführten Mengen resultiert in einem besonders guten Farbschutz und führt nicht zu einer Ausfällung des sauren Proteins oder zu negativen Wechselwirkungen mit weiteren üblichen Inhaltsstoffen des erfindungsgemäßen kosmetischen Mittels, so dass eine hohe Lagerstabilität gewährleistet wird. Darüber hinaus führt der Einsatz dieser Salzmengen auch nicht zu einem negativen Einfluss auf die Reinigungs- bzw. Pflegeleistung der erfindungsgemäßen kosmetischen Mittel.

Chelatisierungs- und/oder Komplexierungsmittel, insbesondere Stickstoff-enthaltende Chelatisierungs- und/oder Komplexierungsmittel, können sich negativ auf die zwingend in den erfindungsgemäßen kosmetischen Mitteln enthaltenden Salze auswirken, indem sie die Kationen der teilweise bis vollständig gelösten Salze komplexieren und damit ihre Wirksamkeit beeinträchtigen. Die erfindungsgemäßen kosmetischen Mittel sind daher bevorzugt im Wesentlichen frei von Stickstoff-enthaltenden Chelatisierungs- und/oder Komplexierungsmitteln, wie beispielsweise β-Alanindiessigsäure, Diethylentriaminpentamethylenphosphonsäure, Natrium-, Kalium-, Calciumdinatrium-, Ammonium- und Triethanolaminsalzen der Ethylendiamintetraessigsäure (EDTA), Hydroxyethylethylendiamintetraessigsäure (HEDTA) und ihren Natriumsalzen, Natriumsalzen der Nitrilotriessigsäure (NTA), Diethylentriaminpentaessigsäure, Aminotrimethylenphosphonat-Pentanatrium, Ethylendiamin-tetramethylenphosphonat-Pentanatrium, Diethylentriamin-pentaacetat-Pentanatrium, Tetra-hydroxyethylethylendiamin, Tetrahydroxypropylethylendiamin, Trinatriumethylendiamindisuccinat, Tetranatrium-N,N-bis(Carboxymethyl)glutamat und Tetranatrium-DL-Alan in-N,N-diacetat.

Unter "im Wesentlichen frei" wird erfindungsgemäß verstanden, dass die kosmetischen Mittel maximal 0,01 Gew.-%, vorzugsweise maximal 0,005 Gew.-%, insbesondere maximal 0,001 Gew.-%, bezogen auf das Gesamtgewicht des kosmetischen Mittels, Stickstoff-enthaltende Chelatisierungs- und/oder Komplexierungsmittel enthalten.

Es wurde gefunden, dass der Farberhalt gefärbter keratinischer Fasern nach mehreren Reinigungsvorgängen besonders hoch ist, wenn die erfindungsgemäßen kosmetischen Mittel einen bestimmten pH-Wert aufweisen. Im Rahmen der vorliegenden Erfindung ist es daher bevorzugt, wenn das kosmetische Mittel einen pH-Wert, gemessen bei 20 °C, von pH 4,0 bis pH 5,0, vorzugsweise von pH 4,1 bis pH 4,9, bevorzugt von pH 4,2 bis pH 4,8, insbesondere von pH 4,3 bis pH 4,7, aufweist.

In einer weiteren bevorzugten Ausführungsform sind die erfindungsgemäßen kosmetischen Mittel dadurch gekennzeichnet, dass sie - bezogen auf das Gesamtgewicht der erfindungsgemäßen kosmetischen Mittel -
- 0,001 bis 10 Gew.-% mindestens eines Proteins, welches einen Gehalt an Glutaminsäure und/oder Asparaginsäure von 10 bis 60 %, vorzugsweise von 20 bis 60 %, bevorzugt von 30 bis 60 %, insbesondere von 40 bis 60 % bezogen auf den Gesamtgehalt aller Aminosäuren des Proteins, aufweist,
- 0,01 bis 10 Gew.-%, vorzugsweise 0,01 bis 5 Gew.-%, bevorzugt von 0,05 bis 4,5 Gew.-%, weiter bevorzugt von 0,1 bis 4,0 Gew.-%, noch weiter 0,15 bis 3,5 Gew.-%, insbesondere von 0,8 bis 2,5 Gew.-% Aluminiumsalze der Formel MAl(SO4)₂, worin M für ein Kaliumion steht,
enthalten,
wobei das kosmetische Mittel einen pH-Wert, gemessen bei 20 °C, von pH 4,0 bis pH 5,0, vorzugsweise von pH 4,1 bis pH 4,9, bevorzugt von pH 4,2 bis pH 4,8, insbesondere von pH 4,3 bis pH 4,7, aufweist.

In einer wiederum weiteren bevorzugten Ausführungsform sind die erfindungsgemäßen kosmetischen Mittel dadurch gekennzeichnet, dass sie - bezogen auf das Gesamtgewicht der erfindungsgemäßen kosmetischen Mittel -
- 0,001 bis 10 Gew.-% mindestens eines Proteins, welches einen Gehalt an Glutaminsäure und/oder Asparaginsäure von 10 bis 60 %, vorzugsweise von 20 bis 60 %, bevorzugt von 30 bis 60 %, insbesondere von 40 bis 60 % bezogen auf den Gesamtgehalt aller Aminosäuren des Proteins, aufweist,
- Calciumlactat und Aluminiumsalze der Formel MAl(SO4)₂, worin M für ein Kaliumion steht, in einer Gesamtmenge von 0,01 bis 10 Gew.-%
enthalten,
wobei das kosmetische Mittel einen pH-Wert, gemessen bei 20 °C, von pH 4,0 bis pH 5,0, vorzugsweise von pH 4,1 bis pH 4,9, bevorzugt von pH 4,2 bis pH 4,8, insbesondere von pH 4,3 bis pH 4,7, aufweist.

Die erfindungsgemäßen kosmetischen Mittel können weitere, für Haarbehandlungsmittel übliche Wirk- und Inhaltsstoffe enthalten.

Bevorzugt enthalten die erfindungsgemäßen kosmetischen Mittel mindestens ein Tensid. Gemäß einer Ausführungsform der vorliegenden Erfindung ist es daher bevorzugt, wenn das kosmetische Mittel zusätzlich mindestens ein Tensid, ausgewählt aus der Gruppe von anionischen Tensiden, kationischen Tensiden, amphoteren Tensiden, nichtionischen Tensiden sowie deren Mischungen, in einer Gesamtmenge von 0,01 bis 20 Gew.-%, bezogen auf das Gesamtgewicht des kosmetischen Mittels, enthält.

Anionische Tenside sind insbesondere dann bevorzugt, wenn das erfindungsgemäße kosmetische Mittel in Form eines Haarshampoos vorliegt. Als anionische Tenside können in den erfindungsgemäßen kosmetischen Mitteln alle für die Verwendung am menschlichen Körper geeigneten anionischen Tenside bzw. oberflächenaktiven Stoffe eingesetzt werden. Diese sind gekennzeichnet durch eine hydrophile anionische Gruppe, wie z. B. eine Carboxylat-, Sulfat-, Sulfonat- oder Phosphatgruppe und eine lipophile Alkylgruppe mit etwa 8 bis 30 Kohlenstoffatomen. Zusätzlich können die Tenside Glykol- oder Polyglykolethergruppen, Ester-, Ether- und Amidgruppen sowie Hydroxylgruppen aufweisen. Zu der Gruppe der im Rahmen der vorliegenden Erfindung einsetzbaren anionischen Tenside zählen beispielsweise:
- Ethercarbonsäuren der Formel R-O-(CH₂-CH₂O)ₓ-CH₂-COOH, in welcher R eine lineare oder verzweigte, gesättigte oder ungesättigte Alkylgruppe mit 8 bis 30 Kohlenstoffatomen und x = 0 oder 1 bis 16 ist,
- Acylglutamat- und/oder (Acyl)isethionate mit 8 bis 24 Kohlenstoffatomen in der Acylgruppe,
- Sulfobernsteinsäuremono- und/oder -dialkylester mit 8 bis 24 Kohlenstoffatomen in der Alkylgruppe und Sulfobernsteinsäuremono-alkylpolyoxyethylester mit 8 bis 24 Kohlenstoffatomen in der Alkylgruppe und 1 bis 6 Oxyethylgruppen, und/oder
- Alkylsulfat- und/oder Alkylpolyglykolethersulfatsalze der Formel (I) R¹-(OCH₂-CH₂)ₓ-OSO₃⁻X⁺, in welcher R¹ bevorzugt für eine lineare oder verzweigte, gesättigte oder ungesättigte Alkylgruppe mit 8 bis 30 Kohlenstoffatomen, x für die Zahl 0 oder 1 bis 12 und X für ein Alkali-, Erdalkali-, Ammonium- oder Alkanolaminion steht.

Besonders bevorzugte anionische Tenside sind geradkettige oder verzweigte Alkylethersulfate der zuvor genannten Formel (I), welche einen Alkylrest mit 8 bis 18, insbesondere mit 10 bis 16 Kohlenstoffatomen, sowie 1 bis 6 und insbesondere 2 bis 4 Ethylenoxideinheiten enthalten. Insbesondere bevorzugt sind die Natrium-, Magnesium und/oder Triethanolaminsalze linearer oder verzweigter Lauryl-, Tridecyl- und/oder Myristylsulfate, welche einen Ethoxylierungsgrad von 2 bis 4 aufweisen.

Die anionischen Tenside werden bevorzugt in bestimmten Gesamtmengen eingesetzt. Bevorzugte erfindungsgemäße kosmetische Mittel sind daher dadurch gekennzeichnet, dass sie mindestens ein anionisches Tensid in einer Gesamtmenge von 0,1 bis 20 Gew.-%, bezogen auf das Gesamtgewicht des kosmetischen Mittels, enthalten.

In diesem Zusammenhang sind erfindungsgemäße kosmetische Mittel bevorzugt, welche - bezogen auf das Gesamtgewicht der erfindungsgemäßen kosmetischen Mittel -
- 0,001 bis 10 Gew.-% mindestens eines Proteins, welches einen Gehalt an Glutaminsäure und/oder Asparaginsäure von 10 bis 60 %, vorzugsweise von 20 bis 60 %, bevorzugt von 30 bis 60 %, insbesondere von 40 bis 60 % bezogen auf den Gesamtgehalt aller Aminosäuren des Proteins, aufweist,
- Calciumlactat und Aluminiumsalze der Formel MAl(SO4)₂, worin M für ein Kaliumion steht, in einer Gesamtmenge von 0,01 bis 10 Gew.-%,
- 0,1 bis 20 Gew.-% mindestens eines anionischen Tensids, ausgewählt aus der Gruppe von Alkylsulfat- und/oder Alkylpolyglykolethersulfatsalze der Formel (I) R¹-(OCH₂-CH₂)ₓ-OSO₃⁻X⁺, in welcher R¹ bevorzugt für eine lineare oder verzweigte, gesättigte oder ungesättigte Alkylgruppe mit 8 bis 30 Kohlenstoffatomen, x für die Zahl 0 oder 1 bis 12 und X für ein Alkali-, Erdalkali-, Ammonium- oder Alkanolaminion steht,
enthalten,
wobei das kosmetische Mittel einen pH-Wert, gemessen bei 20 °C, von pH 4,0 bis pH 5,0, vorzugsweise von pH 4,1 bis pH 4,9, bevorzugt von pH 4,2 bis pH 4,8, insbesondere von pH 4,3 bis pH 4,7, aufweist.

Werden die erfindungsgemäßen kosmetischen Mittel als Pflegespülung konfektioniert, ist der Einsatz von kationischen Tensiden bevorzugt. In diesem Zusammenhang geeignete kationische Tenside sind beispielsweise quartäre Ammoniumverbindungen, Esterquats und/oder Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83 und Quaternium-87 bekannten Imidazolium-Verbindungen. Die Alkylketten der oben genannten Tenside weisen bevorzugt 10 bis 18 Kohlenstoffatome auf.

Bei Esterquats handelt es sich um Stoffe, welche sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart®, Armocare® und Quartamin® vertrieben.

Die Alkylamidoamine werden üblicherweise durch Amidierung natürlicher oder synthetischer Fettsäuren und Fettsäureschnitte mit Dialkylaminoaminen hergestellt. Eine besonders geeignete Verbindung aus dieser Substanzgruppe stellt das unter der Bezeichnung Tegoamid® S 18 im Handel erhältliche Stearamidopropyldimethylamin dar.

Die kationischen Tenside werden bevorzugt in bestimmten Gesamtmengen eingesetzt. Bevorzugte erfindungsgemäße kosmetische Mittel sind daher dadurch gekennzeichnet, dass sie mindestens ein kationisches Tensid in einer Gesamtmenge von 0,1 bis 10 Gew.-%, bezogen auf das Gesamtgewicht des kosmetischen Mittels, enthalten.

Als amphotere Tenside werden solche oberflächenaktiven Verbindungen bezeichnet, welche mindestens eine quartäre Ammoniumgruppe und mindestens eine -COO⁽⁻⁾- oder -SO₃⁽⁻⁾-Gruppe aufweisen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Erfindungsgemäß besonders bevorzugte amphotere Tenside sind das unter der INCI-Bezeichnung Cocamidopropyl Betaine bekannte Carbonsäureamid-Derivat sowie Alkylbetaine mit 10 bis 20 Kohlenstoffatomen in der Alkylgruppe.

Die im Rahmen der vorliegenden Erfindung einsetzbaren nichtionischen Tenside weisen als hydrophile Gruppe z. B. eine Polyolgruppe, eine Polyalkylenglykolethergruppe oder eine Kombination aus Polyol- und Polyglykolethergruppe auf. Zu den erfindungsgemäß geeigneten nichtionischen Tensiden/Emulgatoren zählen bevorzugt
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 Kohlenstoffatomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Fettsäurealkanolamiden der allgemeinen Formel (II) 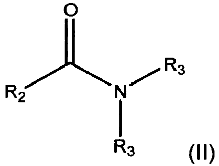 in welcher R₂ bevorzugt einen linearen oder verzweigten, gesättigten oder ungesättigten Alkyl- oder Alkenylrest mit 8 bis 24 Kohlenstoffatomen bedeutet und die Reste R₃ für Wasserstoff oder für die Gruppe -(CH₂)ₙOH stehen, in welcher n für die Zahlen 2 oder 3 steht, mit der Maßgabe, dass mindestens einer der Reste R₃ für den zuvor genannten Rest -(CH₂)ₙOH steht,
- Anlagerungsprodukte von Ethylenoxid an Fettamine und/oder
- Alkyloligoglucoside, insbesondere Alkyloligoglucoside auf der Basis von gehärtetem C_{12/14}-
- Kokosalkohol mit einem DP von 1 bis 3, wie sie beispielsweise unter der INCI-Bezeichnung "Coco-Glucoside", "Decyl Glucoside" und/oder "Lauryl Glucoside" im Handel erhältlich sind.

Die optischen und haptischen Eigenschaften der erfindungsgemäßen kosmetischen Mittel können noch weiter gesteigert werden, wenn Ihnen Haarpflegestoffe hinzugefügt werden. Als geeignete weitere Haarpflegestoffe kommen bevorzugt kosmetisch geeignete Öl-, Fett- und/oder Wachskomponenten und/oder Vitamine in Betracht.

Kosmetisch geeignete Öl- Wachs- und/oder Fettkomponenten können bevorzugt ausgewählt sein aus mineralischen, natürlichen und synthetischen Ölkomponenten und/oder Fettstoffen.

Als natürliche (pflanzliche) Öle werden üblicherweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol® S).

Als Ölkomponente kann weiterhin ein Dialkylether dienen. Einsetzbare Dialkylether sind insbesondere Di-n-alkylether mit insgesamt zwischen 12 bis 36 Kohlenstoffatomen, insbesondere 12 bis 24 Kohlenstoffatomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether. Erfindungsgemäß besonders bevorzugt wird Di-n-octylether, welcher im Handel unter der Bezeichnung Cetiol® OE erhältlich ist, eingesetzt.

Als synthetische Öle kommen bevorzugt Silikonverbindungen in Betracht. Silikone bewirken auf dem Haar ausgezeichnete konditionierende Eigenschaften. Insbesondere wirken sie sich in vielen Fällen positiv auf den Haargriff und die Weichheit der Haare aus. Es ist daher bevorzugt, Silikone in den erfindungsgemäßen kosmetischen Mitteln einzusetzen. Geeignete Silikone werden bevorzugt ausgewählt aus der Gruppe von:
(i) Polyalkylsiloxanen, Polyarylsiloxanen, Polyalkylarylsiloxanen, welche flüchtig oder nicht flüchtig, geradkettig, verzweigt oder cyclisch, vernetzt oder nicht vernetzt sind;
(ii) Polysiloxanen, welche in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) deren Gemischen.

Für die Erhöhung des Haarglanzes und zur Erzielung eines weicheren Haargriffs hat es sich als besonders bevorzugt erwiesen, wenn die erfindungsgemäßen kosmetischen Mittel mindestens ein Silikon enthalten. Erfindungsgemäß bevorzugte kosmetische Mittel sind daher dadurch gekennzeichnet, dass sie zusätzlich mindestens ein Silikon in einer Gesamtmenge von 0,01 bis 3 Gew.-%, bezogen auf das Gesamtgewicht des kosmetischen Mittels, enthalten.

In diesem Zusammenhang sind erfindungsgemäße kosmetische Mittel bevorzugt, welche - bezogen auf das Gesamtgewicht der erfindungsgemäßen kosmetischen Mittel -
- 0,001 bis 10 Gew.-% mindestens eines Proteins, welches einen Gehalt an Glutaminsäure und/oder Asparaginsäure von 10 bis 60 %, vorzugsweise von 20 bis 60 %, bevorzugt von 30 bis 60 %, insbesondere von 40 bis 60 % bezogen auf den Gesamtgehalt aller Aminosäuren des Proteins, aufweist,
- Calciumlactat und Aluminiumsalze der Formel MAl(SO4)₂, worin M für ein Kaliumion steht, in einer Gesamtmenge von 0,01 bis 10 Gew.-%,
- 0,01 bis 3 Gew.-% mindestens eines Silikons,
enthalten,
wobei das kosmetische Mittel einen pH-Wert, gemessen bei 20 °C, von pH 4,0 bis pH 5,0, vorzugsweise von pH 4,1 bis pH 4,9, bevorzugt von pH 4,2 bis pH 4,8, insbesondere von pH 4,3 bis pH 4,7, aufweist.

Unter Fettstoffen sind Fettsäuren, Fettalkohole sowie natürliche und synthetische Wachse zu verstehen, welche sowohl in fester Form als auch flüssig in wässriger Dispersion vorliegen können.

Als Fettsäuren können lineare und/oder verzweigte, gesättigte und/oder ungesättigte Fettsäuren mit 6 bis 30 Kohlenstoffatomen eingesetzt werden. Bevorzugt sind Fettsäuren mit 10 bis 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können gesättigte, ein- oder mehrfach ungesättigte, verzweigte oder unverzweigte Fettalkohole mit C₆ bis C₃₀-, bevorzugt C₁₀ bis C₂₂- und ganz besonders bevorzugt C₁₂ bis C₂₂-Kohlenstoffatomen eingesetzt werden. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, welche durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden.

Als natürliche oder synthetische Wachse können feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP eingesetzt werden. Derartige Wachse sind beispielsweise erhältlich über die Fa. Kahl & Co., Trittau.

Weitere Fettstoffe sind beispielsweise
- Esteröle, also die Ester von C₆ bis C₃₀-Fettsäuren mit C₂ bis C₃₀-Fettalkoholen. Bevorzugt sind die Monoester der Fettsäuren mit Alkoholen mit 2 bis 24 Kohlenstoffatomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit® IPM), Isononansäure-C₁₆-C₁₈-alkylester (Cetiol® SN), 2-Ethylhexylpalmitat (Cegesoft® 24), Stearinsäure-2-ethylhexylester (Cetiol®868), Cetyloleat, Glycerintricaprylat, Kokosfettalkohol- caprinat/- caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Diisotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol® CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls® 90-O18, Monomuls® 90-L12, Cetiol® HE oder Cutina® MD.

Zur Erhöhung der Pflegeeigenschaften können die erfindungsgemäßen kosmetischen Mittel mindestens ein kationisches Polymer enthalten. Unter erfindungsgemäß geeigneten kationischen Polymeren sind Polymere zu verstehen, welche in der Haupt- und/oder Seitenkette "temporär kationische" oder "permanent kationische" Gruppen aufweisen. Als "permanent kationisch" werden erfindungsgemäß solche Polymere bezeichnet, welche unabhängig vom pH-Wert des Mittels eine kationische Gruppe aufweisen. Dies sind in der Regel Polymere, welche ein quartäres Stickstoffatom, beispielsweise in Form einer Ammoniumgruppe, enthalten.

Unter geeigneten kationischen Polymeren sind beispielsweise zu verstehen:
- quaternisierte Cellulosepolymere, vor allem Polyquaternium-10, wie sie unter den Bezeichnungen Celquat® und Polymer JR® im Handel erhältlich sind,
- hydrophob modifizierte Cellulosederivate, beispielsweise die unter dem Handelsnamen SoftCat® vertriebenen kationischen Polymere,
- kationische Alkylpolyglycoside,
- kationisierter Honig, beispielsweise das Handelsprodukt Honeyquat® 50,
- kationische Guar-Derivate, wie insbesondere die unter den Handelsnamen Cosmedia®Guar N-Hance® und Jaguar® vertriebenen Produkte,
- polymere Dimethyldiallylammoniumsalze und deren Copolymere mit Estern und Amiden von Acrylsäure und Methacrylsäure, vor allem Polyquaternium-6 und Polyquaternium-7. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Besonders bevorzugte kationische Polymere sind quaternisierte Cellulosepolymere, hydrophob modifizierte quaternisierte Cellulosepolymere, kationische Guarderivate und/oder kationische Polymere auf Acrylsäure(derivat)basis, welche besonders bevorzugt ausgewählt sind aus den unter den INCI-Bezeichnungen bekannten Polymeren Guar Hydroxypropyltrimonium Chloride, Polyquaternium-6, Polyquaternium-7, Polyquaternium-10, Polyquaternium-37 und/oder Polyquaternium-67.

Als ganz besonders bevorzugte kationische Polymere für die erfindungsgemäßen kosmetischen Mittel haben sich kationische Polysaccharidpolymere, insbesondere quaternisierte Cellulosepolymere, herausgestellt.

Um einen zufriedenstellenden Pflegeeffekt zu gewährleisten, enthält das erfindungsgemäße kosmetische Mittel bevorzugt zusätzlich mindestens ein kationisches Polymer in einer Gesamtmenge von 0,01 bis 3 Gew.-%, bezogen auf das Gesamtgewicht des kosmetischen Mittels.

In diesem Zusammenhang sind erfindungsgemäße kosmetische Mittel bevorzugt, welche - bezogen auf das Gesamtgewicht der erfindungsgemäßen kosmetischen Mittel -
- 0,001 bis 10 Gew.-% mindestens eines Proteins, welches einen Gehalt an Glutaminsäure und/oder Asparaginsäure von 10 bis 60 %, vorzugsweise von 20 bis 60 %, bevorzugt von 30 bis 60 %, insbesondere von 40 bis 60 % bezogen auf den Gesamtgehalt aller Aminosäuren des Proteins, aufweist,
- Calciumlactat und Aluminiumsalze der Formel MAl(SO₄)₂, worin M für ein Kaliumion steht, in einer Gesamtmenge von 0,01 bis 10 Gew.-%,
- 0,01 bis 3 Gew.-% mindestens eines kationisches Polymers, ausgewählt aus der Gruppe von kationischen Polysacchariden,
enthalten,
wobei das kosmetische Mittel einen pH-Wert, gemessen bei 20 °C, von pH 4,0 bis pH 5,0, vorzugsweise von pH 4,1 bis pH 4,9, bevorzugt von pH 4,2 bis pH 4,8, insbesondere von pH 4,3 bis pH 4,7, aufweist.

Unter geeigneten Vitaminen sind bevorzugt die folgenden Vitamine, Provitamine und Vitaminvorstufen sowie deren Derivate zu verstehen:
- Vitamin A, wie beispielsweise das Retinol (Vitamin A₁) sowie das 3,4-Didehydroretinol (Vitamin A₂), das β-Carotin, Vitamin A-Säure und deren Ester, Vitamin A-Aldehyd und Vitamin A-Alkohol sowie dessen Ester,
- Vitamin B, wie beispielsweise das Vitamin B₁ (Thiamin), das Vitamin B₂ (Riboflavin) sowie das Vitamin B₃ (Nicotinsäure oder Nicotinsäureamid (Niacinamid)),
- Vitamin B₅ (Pantothensäure und Panthenol) sowie dessen Derivate, insbesondere die Ester und Ether des Panthenols, Pantolacton sowie kationisch derivatisierte Panthenole,
- Vitamin B₆ (Pyridoxin sowie Pyridoxamin und Pyridoxal),
- Vitamin C (Ascorbinsäure), insbesondere in Form des Palmitinsäureesters, der Glucoside oder Phosphate,
- Vitamin E (Tocopherole, insbesondere α-Tocopherol),
- Vitamin F, wie beispielsweise Linolsäure, Linolensäure und Arachidonsäure, sowie
- Vitamin H ((3aS,4S, 6aR)-2-Oxohexahydrothienol[3,4-d]-imidazol-4-valeriansäure bzw. Biotin).

Besonders bevorzugt sind Vitamine, Provitamine und Vitaminvorstufen aus den Gruppen A, B, E und H. Ganz besonders bevorzugt sind Nicotinsäureamid, Biotin, Pantolacton und/oder Panthenol. Insbesondere bevorzugt ist Panthenol.

Das/die Vitamin(e), Vitaminderivat(e), und/oder Vitaminvorstufe(n) werden in den erfindungsgemäßen kosmetischen Mittel bevorzugt in einer Gesamtmenge von 0,001 bis 2 Gew.-%, vorzugsweise von 0,005 bis 1 Gew.-%, insbesondere von 0,01 bis 0,5 Gew.-%, bezogen auf das Gesamtgewicht des kosmetischen Mittels, eingesetzt.

Neben den erfindungsgemäß zwingenden Komponenten und den weiteren, oben genannten bevorzugten Komponenten können prinzipiell alle weiteren, dem Fachmann für solche kosmetischen Zusammensetzungen bekannten Komponenten eingesetzt werden.

Weitere Wirk-, Hilfs- und Zusatzstoffe sind beispielsweise:
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, vollsynthetische Hydrokolloide wie z. B. Polyvinylalkohol,
- Strukturanten wie Maleinsäure und Milchsäure,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- weitere Substanzen zur Einstellung des pH-Wertes, wie beispielsweise α- und β-Hydroxycarbonsäuren,
- Wirkstoffe wie Allantoin und Bisabolol,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Viskositätsregler wie Salze (NaCl).

Ein weiterer Gegenstand der vorliegenden Erfindung betrifft ein Verfahren zum Farberhalt gefärbter keratinischer Fasern, bei welchem ein erfindungsgemäßes kosmetisches Mittel innerhalb eines Zeitraums von 5 Sekunden bis 24 Stunden nach Färbung der keratinischen Fasern auf diese Fasern aufgebracht und gegebenenfalls nach einer Einwirkzeit von 30 Sekunden bis 30 Minuten aus den keratinischen Fasern ausgespült wird.

Unter Farberhalt wird in diesem Zusammenhang der Schutz der ursprünglichen Farbe der gefärbten keratinischen Fasern verstanden. Insbesondere soll eine Farbverschiebung zu gelblichen bzw. bläulichen Farbtönen während der Reinigung bzw. Pflege der keratinischen Fasern vermieden werden.

Weiterhin wird unter dem Begriff "Färbung" im Rahmen der vorliegenden Erfindung die künstliche Veränderung der Haarfarbe, insbesondere unter Verwendung von Oxidationsfärbemitteln oder temporären Färbemittel, verstanden.

In bevorzugten erfindungsgemäßen Verfahren werden die kosmetischen Mittel innerhalb einer kürzeren Zeit nach der Färbung aufgebracht. Erfindungsgemäß bevorzugte Verfahren sind daher dadurch gekennzeichnet, dass das kosmetische Mittel innerhalb eines Zeitraums von 5 Sekunden bis 20 Stunden, vorzugsweise von 10 Sekunden bis 16 Stunden, bevorzugt von 30 Sekunden bis 14 Stunden, insbesondere von 1 Minute bis 12 Stunden, nach der Färbung auf die keratinischen Fasern aufgebracht wird.

Bezüglich weiterer Ausführungsformen des erfindungsgemäßen Verfahrens sowie bezüglich der in dem erfindungsgemäßen Verfahren eingesetzten kosmetischen Mittel gilt mutatis mutandis das zu den erfindungsgemäßen kosmetischen Mitteln Gesagte.

Schließlich ist ein weiterer Gegenstand der vorliegenden Erfindung die Verwendung einer Kombination aus
a) 0,001 bis 10 Gew.-% mindestens eines Proteins, welches einen Gehalt an Glutaminsäure und/oder Asparaginsäure von 10 bis 60 %, bezogen auf den Gesamtgehalt aller Aminosäuren des Proteins, aufweist, und
b) 0,01 bis 10 Gew.-% mindestens eines Salzes, welches mindestens ein zweiwertiges Kation umfasst, und/oder mindestens eines Salzes, welches mindestens ein dreiwertiges Kation umfasst,
wobei als Salz, welches mindestens ein dreiwertiges Kation umfasst, ein Salz aus der Gruppe von Aluminiumsalzen der Formel MAl(SO₄)₂, worin M für ein Kalium-, Natrium-, Guanidinium- oder Ammoniumion steht, enthalten ist,
zum Farberhalt gefärbter keratinischer Fasern.

Durch die Verwendung der zuvor angeführten Kombination aus einem sauren Protein und mindestens einem Salz kann der Farbverlust bei der Reinigung und/oder Pflege von gefärbten keratinischen Fasern vermieden werden. Darüber hinaus wird durch Verwendung der zuvor angeführten Kombination auch die Farbverschiebung während der Reinigung und/oder Pflege von gefärbten keratinischen Fasern vermindert. Auf diese Weise bleibt die nach der Haarfärbung erhaltene Farbe länger unverändert erhalten.

Bezüglich weiterer Ausführungsformen der erfindungsgemäßen Verwendung gilt mutatis mutandis das zu den erfindungsgemäßen kosmetischen Mitteln sowie zu dem erfindungsgemäßen Verfahren Gesagte.

Die nachfolgenden Beispiele sollen bevorzugte Ausführungsformen der Erfindung erläutern, ohne sie jedoch einzuschränken.

### Beispiele:

Es wurden die folgenden erfindungsgemäßen kosmetischen Mittel hergestellt (die Mengenangaben beziehen sich dabei auf Gew.-%):

**a) Farbschutz-Shampoos**

| | **E1** Vergleich | **E2** Vergleich | **E3** | **E4** |
|---|---|---|---|---|
| Sodium Laureth Sulfate | 11,00 | 11,00 | 11,00 | 11,00 |
| Cocamidopropyl Betaine | 1,00 | 1,50 | 1,50 | 1,50 |
| Disodium Cocoamphodiacetate | 0,50 | -- | -- | -- |
| Cocamide MEA | 0,50 | 0,50 | 0,50 | 0,50 |
| PEG-12 Dimethicone | 0,50 | 0,30 | 0,30 | 0,30 |
| Glycol Distearate | 1,20 | -- | -- | -- |
| PEG-7 Glyceryl Cocoate | 0,40 | 0,60 | 0,60 | 0,60 |
| Polyquaternium-10 | 0,90 | 0,60 | 0,60 | 0,60 |
| Panthenol | 0,30 | 0,20 | 0,20 | 0,20 |
| Aprikosenkernöl | 0,05 | -- | -- | -- |
| Hydrogenated Castor Oil | 0,20 | 0,10 | 0,10 | 0,10 |
| Protein mit mindestens 10 bis 60 % Asparaginsäure und/oder Glutaminsäure | 0,5 | 1,0 | 1,0 | 1,0 |
| Calcium lactat | 0,2 | 0,5 | -- | 0,2 |
| KAl(SO₄)₂ | -- | -- | 1,0 | 0,8 |
| Konservierungsmittel, Parfum, evtl. Säuerungsmittel | q.s. | q.s. | q.s. | q.s. |
| Wasser | ad 100 | ad 100 | ad 100 | ad 100 |
| pH | 4,5 | 4,4 | 4,4 | 4,4 |

| | | | | |
|---|---|---|---|---|
| Die Shampoos E1 bis E4 wurden auf angefeuchtete gefärbte Haare aufgetragen und nach einer Einwirkungszeit von etwa einer Minute mit Wasser ausgespült. Die Haare wiesen nach der Reinigung eine brillante Haarfarbe auf, welche sich nicht verändert hatte. Zudem wiesen sie einen optisch ansprechenden Glanz auf und fühlten sich weich an. | | | | |

**b) Conditioner**

| | **E4** Vergleich | **E5** Vergleich | **E6** | **E7** |
|---|---|---|---|---|
| Cetearyl Alcohol | 2,50 | 2,50 | 2,50 | 2,50 |
| Quaternium-87 | 2,00 | 2,00 | 2,00 | 2,00 |
| Propylene Glycol | 0,50 | 0,50 | 0,50 | 0,50 |
| Isopropyl Myristate | 0,30 | 0,30 | 0,30 | 0,30 |
| Distearoylethyl Hydroxyethylmonium Methosulfate | 0,50 | 0,50 | 0,50 | 0,50 |
| Calcium Hydroxide | 0,54 | -- | -- | -- |
| Phenoxyethanol | 0,40 | 0,40 | 0,40 | 0,40 |
| Stearamidopropyl Dimethylamine | 0,30 | 0,30 | 0,30 | 0,30 |
| Parfum | 0,25 | 0,25 | 0,25 | 0,25 |
| Sodium Methylparaben | 0,20 | 0,20 | 0,20 | 0,20 |
| Polyquaternium-37 | 0,20 | 0,20 | 0,20 | 0,20 |
| Dicaprylyl Carbonate | 0,20 | 0,20 | 0,20 | 0,20 |
| Panthenol | 0,10 | 0,10 | 0,10 | 0,10 |
| Benzophenone-4 | 0,05 | 0,05 | 0,05 | 0,05 |
| Amodimethicone/Morpholinomethyl Silsesquioxane Copolymer | 0,02 | 0,02 | 0,02 | 0,02 |
| Glycerin | 0,01 | 0,01 | 0,01 | 0,01 |
| Hydrolyzed Keratin | 0,01 | 0,01 | 0,01 | 0,01 |
| Protein mit mindestens 10 bis 60 % Asparaginsäure und/oder Glutaminsäure | 0,5 | 1,0 | 1,0 | 1,0 |
| Calciumlactat | 0,2 | 0,5 | -- | 0,2 |
| KAl(SO₄)₂ | -- | -- | 1,0 | 0,8 |
| Konservierungsmittel, Parfum, evtl. Säuerungsmittel | q.s. | q.s. | q.s. | q.s. |
| Wasser | ad 100 | ad 100 | ad 100 | ad 100 |
| pH | 4,3 | 4,3 | 4,3 | 4,3 |

| | | | | |
|---|---|---|---|---|
| Die Conditioner E4 bis E7 wurden auf angefeuchtete gefärbte Haare aufgetragen und nach einer Einwirkungszeit von etwa einer Minute mit Wasser ausgespült. Die Haare wiesen nach der Reinigung eine brillante Haarfarbe auf, welche sich nicht verändert hatte. Zudem wiesen sie einen optisch ansprechenden Glanz auf und fühlten sich weich an. | | | | |

## Patentansprüche

1. Kosmetisches Mittel zur Behandlung keratinischer Fasern, enthaltend in einem kosmetisch verträglichen Träger - bezogen auf das Gesamtgewicht des kosmetischen Mittels -
a) 0,001 bis 10 Gew.-% mindestens eines Proteins, welches einen Gehalt an Glutaminsäure und/oder Asparaginsäure von 10 bis 60 %, bezogen auf den Gesamtgehalt aller Aminosäuren des Proteins, aufweist, und
b) 0,01 bis 10 Gew.-% mindestens eines Salzes, welches mindestens ein zweiwertiges Kation umfasst, und/oder mindestens eines Salzes, welches mindestens ein dreiwertiges Kation umfasst,
**dadurch gekennzeichnet, dass** als Salz, welches mindestens ein dreiwertiges Kation umfasst, ein Salz aus der Gruppe von Aluminiumsalzen der Formel MAl(SO₄)₂, worin M für ein Kalium-, Natrium-, Guanidinium- oder Ammoniumion steht, enthalten ist.

2. Kosmetisches Mittel nach Anspruch 1, **dadurch gekennzeichnet, dass** das mindestens eine Protein einen Gehalt an Glutaminsäure und/oder Asparaginsäure von 20 bis 60 %, vorzugsweise von 30 bis 60 %, insbesondere von 40 bis 60 %, bezogen auf den Gesamtgehalt aller Aminosäuren des Proteins, aufweist.

3. Kosmetisches Mittel nach einem der Ansprüche 1 oder 2, **dadurch gekennzeichnet, dass** das mindestens eine Protein einen pKₛ-Wert von 3,5 bis 6,5, vorzugsweise von 4,0 bis 6,0, bevorzugt von 4,5 bis 6,0, insbesondere von 4,5 bis 5,5, aufweist.

4. Kosmetisches Mittel nach einem der vorangehenden Ansprüche, **dadurch gekennzeichnet, dass** das mindestens eine Protein in einer Gesamtmenge von 0,001 bis 8 Gew.-%, vorzugsweise von 0,01 bis 5 Gew.-%, bevorzugt von 0,1 bis 3 Gew.-%, insbesondere von 0,3 bis 2 Gew.-%, bezogen auf das Gesamtgewicht des kosmetischen Mittels, enthalten ist.

5. Kosmetisches Mittel nach einem der vorangehenden Ansprüche, **dadurch gekennzeichnet, dass** als Salz, welches mindestens ein zweiwertiges Kation umfasst, ein Salz der Milchsäure, ausgewählt aus der Gruppe von Magnesiumsalzen, Calciumsalzen, Kupfersalzen, Zinksalzen, Eisen(II)-salzen sowie Mischungen dieser Salze, insbesondere Calciumsalzen der Milchsäure, enthalten ist.

6. Kosmetisches Mittel nach einem der vorangehenden Ansprüche, **dadurch gekennzeichnet, dass** das Salz, welches mindestens ein zweiwertiges Kation umfasst, in einer Gesamtmenge von 0,01 bis 8 Gew.-%, vorzugsweise von 0,1 bis 5 Gew.-%, bevorzugt von 0,2 bis 3 Gew.-%, insbesondere von 0,3 bis 2 Gew.-%, bezogen auf das Gesamtgewicht des kosmetischen Mittels, enthalten ist.

7. Kosmetisches Mittel nach einem der vorangehenden Ansprüche, **dadurch gekennzeichnet, dass** das Salz, welches mindestens ein dreiwertiges Kation umfasst, in einer Gesamtmenge von 0,01 bis 5 Gew.-%, vorzugsweise von 0,05 bis 4,5 Gew.-%, bevorzugt von 0,1 bis 4,0 Gew.-%, weiter bevorzugt von 0,15 bis 3,5 Gew.-%, noch weiter bevorzugt von 0,5 bis 3,0 Gew.-%, insbesondere von 0,8 bis 2,5 Gew.-%, bezogen auf das Gesamtgewicht des kosmetischen Mittels, enthalten ist.

8. Verfahren zum Farberhalt gefärbter keratinischer Fasern, bei welchem ein kosmetisches Mittel nach einem der Ansprüche 1 bis 7 innerhalb eines Zeitraums von 5 Sekunden bis 24 Stunden nach Färbung der keratinischen Fasern auf diese Fasern aufgebracht und gegebenenfalls nach einer Einwirkzeit von 30 Sekunden bis 30 Minuten aus den keratinischen Fasern ausgespült wird.

9. Verwendung einer Kombination aus
a) 0,001 bis 10 Gew.-% mindestens eines Proteins, welches einen Gehalt an Glutaminsäure und/oder Asparaginsäure von 10 bis 60 %, bezogen auf den Gesamtgehalt aller Aminosäuren des Proteins, aufweist, und
b) 0,01 bis 10 Gew.-% mindestens eines Salzes, welches mindestens ein zweiwertiges Kation umfasst, und/oder mindestens eines Salzes, welches mindestens ein dreiwertiges Kation umfasst, wobei
als Salz, welches mindestens ein dreiwertiges Kation umfasst, ein Salz aus der Gruppe von Aluminiumsalzen der Formel MAl(SO₄)₂, worin M für ein Kalium-, Natrium-, Guanidinium- oder Ammoniumion steht, enthalten ist,
zum Farberhalt gefärbter keratinischer Fasern.

## Claims

1. A cosmetic agent for the treatment of keratin fibers, containing, in a cosmetically acceptable carrier, based on the total weight of the cosmetic agent,
a) 0.001 to 10 wt.% of at least one protein having a content of glutamic acid and/or aspartic acid of 10 to 60%, based on the total content of all the amino acids of the protein, and
b) 0.01 to 10 wt.% of at least one salt comprising at least one divalent cation, and/or at least one salt which comprises at least one trivalent cation,
**characterized in that** a salt from the group of aluminum salts of the formula MAl(SO₄)₂, in which M represents a potassium, sodium, guanidinium or ammonium ion, is contained as the salt comprising at least one trivalent cation.

2. The cosmetic agent according to claim 1, **characterized in that** the at least one protein has a content of glutamic acid and/or aspartic acid of from 20 to 60%, preferably from 30 to 60%, in particular from 40 to 60%, based on the total content of all the amino acids of the protein.

3. The cosmetic agent according to one of claims 1 or 2, **characterized in that** the at least one protein has a pKₛ value of from 3.5 to 6.5, preferably from 4.0 to 6.0, more preferably from 4.5 to 6.0, in particular from 4.5 to 5.5.

4. The cosmetic agent according to one of the preceding claims, **characterized in that** the at least one protein is contained in a total amount of from 0.001 to 8 wt.%, preferably from 0.01 to 5 wt.%, more preferably from 0.1 to 3 wt.%, in particular from 0.3 to 2 wt.%, based on the total weight of the cosmetic agent.

5. The cosmetic agent according to one of the preceding claims, **characterized in that** a salt of lactic acid, selected from the group of magnesium salts, calcium salts, copper salts, zinc salts, iron (II) salts, and mixtures of these salts, in particular calcium salts of lactic acid, is contained as the salt comprising at least one divalent cation.

6. The cosmetic agent according to one of the preceding claims, **characterized in that** the salt comprising at least one divalent cation alkaline is contained in a total amount of from 0.01 to 8 wt.%, preferably from 0.1 to 5 wt.%, more preferably from 0.2 to 3 wt.%, in particular from 0.3 to 2 wt.%, based on the total weight of the cosmetic agent.

7. The cosmetic agent according to one of the preceding claims, **characterized in that** the salt comprising at least one trivalent cation alkaline is contained in a total amount of from 0.01 to 5 wt.%, preferably from 0.05 to 4.5 wt.%, more preferably from 0.1 to 4.0 wt.%, even more preferably from 0.15 to 3.5 wt.%, yet more preferably from 0.5 to 3.0 wt.%, in particular from 0.8 to 2.5 wt.%, based on the total weight of the cosmetic agent.

8. A method for the color retention of dyed keratin fibers, in which a cosmetic agent according to one of claims 1 to 7 is applied to the keratin fibers within a period of 5 seconds to 24 hours after said fibers have been dyed and is optionally rinsed out of the keratin fibers after a contact time of 30 seconds to 30 minutes.

9. The use of a combination of
a) 0.001 to 10 wt.% of at least one protein having a content of glutamic acid and/or aspartic acid of from 10 to 60%, based on the total content of all the amino acids of the protein, and
b) 0.01 to 10 wt.% of at least one salt comprising at least one divalent cation, and/or at least one salt which comprises at least one trivalent cation, wherein
a salt from the group of aluminum salts of the formula MAl(SO₄)₂, in which M represents a potassium, sodium, guanidinium or ammonium ion, is contained as the salt comprising at least one trivalent cation,
for the color retention of dyed keratin fibers.

## Revendications

1. Agent cosmétique pour le traitement des fibres kératiniques, contenant, dans un support cosmétiquement acceptable - par rapport au poids total du produit cosmétique -
a) de 0,001 à 10 % en poids d'au moins une protéine présentant une teneur en acide glutamique et/ou en acide aspartique comprise entre 10 et 60 %, par rapport à la teneur totale de tous les acides aminés de la protéine, et
b) de 0,01 à 10 % d'au moins un sel comprenant au moins un cation divalent et/ou d'au moins un sel comprenant au moins un cation trivalent,
**caractérisé en ce que** le sel qui comprend au moins un cation trivalent est un sel du groupe des sels d'aluminium de formule MAl(SO₄)₂, dans laquelle M est un ion potassium, sodium, guanidinium ou ammonium.

2. Agent cosmétique selon la revendication 1, **caractérisé en ce que** l'au moins une protéine présente une teneur en acide glutamique et/ou en acide aspartique de 20 à 60 %, de préférence de 30 à 60 %, en particulier de 40 à 60 %, par rapport à la teneur totale de tous les acides aminés de la protéine.

3. Agent cosmétique selon l'une quelconque des revendications 1 ou 2, **caractérisée en ce que** l'au moins une protéine présente une valeur de pKₛ de 3,5 à 6,5, de préférence de 4,0 à 6,0, de manière préférée de 4,5 à 6,0, en particulier de 4,5 à 5,5.

4. Agent cosmétique selon l'une quelconque des revendications précédentes, **caractérisé en ce que** l'au moins une protéine est présente en une quantité totale de 0,001 à 8 % en poids, de préférence de 0,01 à 5 % en poids, de manière préférée de 0,1 à 3 % en poids, en particulier de 0,3 à 2 % en poids, par rapport au poids total de la composition cosmétique.

5. Agent cosmétique selon l'une quelconque des revendications précédentes, **caractérisé en ce qu'**un sel d'acide lactique choisi dans le groupe des sels de magnésium, des sels de calcium, des sels de cuivre, des sels de zinc, des sels de fer (II) ainsi que des mélanges de ces sels, en particulier des sels de calcium d'acide lactique, est présent en tant que sel contenant au moins un cation divalent.

6. Agent cosmétique selon l'une quelconque des revendications précédentes, **caractérisée en ce que** le sel comprenant au moins un cation divalent est présent en une quantité totale de 0,01 à 8 % en poids, de préférence de 0,1 à 5 % en poids, de manière préférée de 0,2 à 3 % en poids, en particulier de 0,3 à 2 % en poids, par rapport au poids total de la composition cosmétique.

7. Agent cosmétique selon l'une des revendications précédentes, **caractérisé en ce que** le sel comprenant au moins un cation trivalent est présent en une quantité totale de 0,01 à 5 % en poids, de préférence de 0,05 à 4,5 % en poids, de manière préférée de 0,1 à 4,0 % en poids, de manière davantage préférée de 0,15 à 3,5 % en poids, de manière davantage préférée encore de 0,5 % à 3,0 % en poids, en particulier de 0,8 à 2,5 % en poids, par rapport au poids total de la composition cosmétique.

8. Procédé de conservation de couleurs de fibres kératiniques colorées, dans lequel une composition cosmétique selon l'une quelconque des revendications 1 à 7 est appliquée sur ces fibres dans une période de 5 secondes à 24 heures après la teinture des fibres kératiniques, et est éventuellement rincée des fibres kératiniques après un temps de contact de 30 secondes à 30 minutes.

9. Utilisation d'une combinaison constituée
a) de 0,001 à 10 % en poids d'au moins une protéine présentant une teneur en acide glutamique et/ou en acide aspartique de 10 à 60 %, par rapport à la teneur totale de tous les acides aminés de la protéine, et
b) de 0,01 à 10 % en poids d'au moins un sel qui comprend au moins un cation divalent, et/ou d'au moins un sel qui comprend au moins un cation trivalent,
un sel du groupe des sels d'aluminium de formule MAl(SO₄)₂, où M est un ion potassium, sodium, guanidinium ou ammonium, étant présent en tant que sel contenant au moins un cation trivalent pour la conservation de la couleur des fibres kératiniques colorées.
